# EUROPÄISCHE PATENTANMELDUNG

(11) **EP 4 174 104 A1**
(43) Veröffentlichungstag der Anmeldung: **03.05.2023**
(21) Anmeldenummer: 21205681.6
(22) Anmeldetag: 29.10.2021
(51) Int. Cl.: C08G 59/62, C07D 307/00

(54) **MANNICHBASE MIT HOHEM ERNEUERBAREM KOHLENSTOFFANTEIL**

(71) Anmelder: Sika Technology AG, 6340 Baar (CH)
(72) Erfinder: Kasemi, Edis, 8046 Zürich (CH); Stadelmann, Ursula, 8046 Zürich (CH); Kramer, Andreas, 8008 Zürich (CH); Burckhardt, Urs, 8049 Zürich (CH)
(74) Vertreter: Sika Patent Attorneys

(57) **Zusammenfassung**

Gegenstand der Erfindung ist eine Mannichbase der Formel (I) sowie ihre Verwendung als Bestandteil eines Härters für Epoxidharze. Die Mannichbase der Formel (I) verfügt über einen hohen Renewable Carbon Index. Sie ermöglicht hochwertige und nachhaltige Epoxidharz-Produkte mit überraschend guter Verarbeitbarkeit, die bei Umgebungstemperatur schnell und störungsfrei aushärten und eine schöne Oberfläche bei hoher Endhärte ausbilden.

## Beschreibung

### Technisches Gebiet

Die Erfindung betrifft Mannichbasen mit einem hohen Anteil an erneuerbarem Kohlenstoff und deren Verwendung zum Aushärten von Epoxidharzen.

### Stand der Technik

Amine werden in der Industrie und im Bauwesen unter anderem als Härter für Epoxidharz-Zusammensetzungen verwendet. Dabei sind je nach Anwendung Eigenschaften gefragt wie eine gute Verarbeitbarkeit und eine schnelle und störungsfreie Aushärtung bei Umgebungstemperaturen zu Produkten von hoher Härte bei geringer Sprödigkeit und mit ebenmässiger Oberfläche ohne Blushingbedingte Trübungen, Flecken oder Krater.

Heutzutage werden vermehrt nachhaltige Epoxidharz-Produkte nachgefragt, insbesondere solche mit einem hohen Gehalt an Rohstoffen aus erneuerbaren Quellen, also Epoxidharz-Produkte, welche zu einem hohen Anteil biobasiert sind. Es besteht somit ein Bedarf nach nachhaltigen Aminhärtern. Ein gebräuchliches Mass für die Nachhaltigkeit von chemischen Rohstoffen ist der Renewable Carbon Index (RCI), welcher den Kohlenstoffanteil aus erneuerbaren Quellen angibt. Er ergibt sich durch Division der Anzahl Kohlenstoffatome aus einer erneuerbaren Quelle mit der gesamten Anzahl Kohlenstoffatome im Rohstoff.

Mannichbasen sind Amingruppen-haltige Kondensationsprodukte von Phenolen mit Aminen und Aldehyden wie insbesondere Formaldehyd. In Epoxidharz-Produkten können sie als alleinige Härter oder als Cohärter und/oder Beschleuniger eingesetzt werden. Besonders nachhaltige Mannichbasen aus dem Stand der Technik sind die auch als Phenalkamine bezeichneten Mannichbasen, bei welchen als Phenolverbindung Cardanol eingesetzt wird, ein aus dem Öl der Cashewnussschale gewonnenes Alkenylphenol-Gemisch mit einem RCI von 1. Mannichbasen mit einem gewissen Gehalt an Kohlenstoffen aus erneuerbaren Quellen sind bekannt, beispielsweise aus US 6,262,148 oder US 2017/0240691. Die Verarbeitbarkeit der damit erhaltenen Epoxidharz-Produkte ist aber aufgrund der hohen Viskosität noch verbesserungsfähig.

### Darstellung der Erfindung

Aufgabe der vorliegenden Erfindung ist es daher, eine Mannichbase zur Verfügung zu stellen, welche geeignet ist zum Aushärten von Epoxidharzen bei Umgebungstemperaturen und eine hohe Verdünnungswirkung auf das Epoxidharz ausübt, um auch bei tiefen Umgebungstemperaturen eine gute Verarbeitung und einen hohen Füllgrad zu ermöglichen.

Überraschenderweise wird diese Aufgabe mit einer Mannichbase der Formel (I) wie in Anspruch 1 beschrieben gelöst. Die erfindungsgemässe Mannichbase enthält eine oder mehrere Furfurylaminogruppen und ist in einem einfachen Verfahren herstellbar, insbesondere durch Kondensation eines Phenols mit einem furfurylierten Amin der Formel (IV) und einem Aldehyd, insbesondere Formaldehyd (Mannichreaktion), oder durch Umaminierung bestehender Mannichbasen, insbesondere solchen mit Dimethylaminomethylgruppen. Die Mannichbase der Formel (I) verfügt über einen hohen Renewable Carbon Index (RCI), wobei insbesondere die Furfurylgruppen biobasiert sind und - je nach Quelle des dem furfurylierten Amin zugrunde liegenden primären Diamins - gegebenenfalls auch dessen C-Atome. Falls der Mannichbase der Formel (I) eine auf nachwachsenden Rohstoffen basierende Phenolverbindung wie Cardanol oder Guajacol zugrunde liegt, verfügt die Mannichbase über einen besonders hohen RCI im Bereich von 0.7 bis 1.

Die Mannichbase der Formel (I) ermöglicht überraschend niedrigviskose Epoxidharz-Produkte mit guter Verarbeitbarkeit und - im Fall von gefüllten Produkten - hohem Füllstoffgehalt, wobei keine oder nur geringe Mengen an weiteren Verdünnern oder Lösemitteln benötigt werden und somit sehr niedrige VOC-Gehalte erreichbar sind. Weiterhin ermöglicht die Mannichbase der Formel (I) eine überraschend schnelle und störungsfreie Aushärtung zu hochwertigen Epoxidharz-Produkten mit hoher Endhärte und hohem Glanz, auch bei kalten Umgebungstemperaturen wie insbesondere 8 °C. Zudem ermöglicht die Mannichbase der Formel (I) sehr geruchsarme Epoxidharz-Produkte, während der Stand der Technik oft eingesetzte Mannichbase 2,4,6-Tris(dimethylaminomethyl)phenol auch bei geringer Einsatzmenge Epoxidharz-Produkt mit starkem, unangenehmem Amingeruch zur Folge hat.

Die Mannichbase der Formel (I) ist besonders geeignet als Härter und/oder Beschleuniger für Epoxidharz-Produkte, insbesondere Beschichtungen oder Formkörper. Insbesondere ermöglicht die Mannichbase der Formel (I) geruchsarme, bei Umgebungstemperaturen schnell und zuverlässig aushärtende Beschichtungen mit schönen, glänzenden Oberflächen ohne durch Blushing verursachte Flecken, Trübungen oder Krater.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist eine Mannichbase der Formel (I), 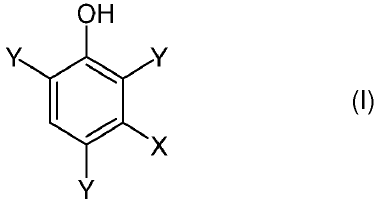 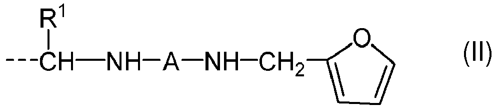 wobei
X für H oder einen linearen Kohlenwasserstoffrest mit 15 C-Atomen steht,
Y unabhängig voneinander jeweils für einen Rest der Formel (II) oder H oder Dimethylaminomethyl oder Methoxy steht, wobei mindestens einer der Reste Y für einen Rest der Formel (II) steht,
R¹ für H oder einen Alkylrest mit 1 bis 6 C-Atomen oder einen Phenylrest steht, und
A für einen Alkylenrest mit 2 bis 10 C-Atomen steht, wobei die beiden Stickstoffatome, an welche der Rest A gebunden ist, durch mindestens zwei C-Atome voneinander getrennt sind.

Eine gestrichelte Linie in den Formeln in diesem Dokument stellt jeweils die Bindung zwischen einem Substituenten und dem zugehörigen Molekülrest dar.

Als "RCI" wird der "Renewable Carbon Index" einer Substanz oder einer Mischung von Substanzen bezeichnet, wobei der RCI für das Verhältnis der Anzahl C-Atome aus biobasierter Quelle zur gesamten Anzahl C-Atome der Substanz oder der Mischung von Substanzen steht.
Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.
Als "Aminwasserstoff" werden die Wasserstoffatome von primären und sekundären Amingruppen bezeichnet.
Als "Aminwasserstoff-Equivalentgewicht" wird die Masse eines Amins oder einer Amin-haltigen Zusammensetzung, die ein Molequivalent Aminwasserstoff enthält, bezeichnet. Es wird angegeben in der Masseinheit "g/eq".
Als "Epoxid-Equivalentgewicht" wird die Masse einer Epoxidgruppen-haltigen Verbindung oder Zusammensetzung bezeichnet, die ein Mol-Equivalent Epoxidgruppen enthält. Es wird angegeben in der Masseinheit "g/eq".
Mit "Poly" beginnende Substanznamen wie Polyamin oder Polyepoxid bezeichnen Substanzen, die formal zwei oder mehr der in ihrem Namen vorkommenden funktionellen Gruppen pro Molekül enthalten.
Als "Verdünner" wird eine in einem Epoxidharz lösliche und dessen Viskosität senkende Substanz bezeichnet, welche bei der Aushärtung chemisch nicht in das Epoxidharz-Polymer eingebunden wird.
Als "Molekulargewicht" wird die molare Masse (in Gramm pro Mol) eines Moleküls bezeichnet. Als "mittleres Molekulargewicht" wird das Zahlenmittel Mₙ einer polydispersen Mischung von oligomeren oder polymeren Molekülen bezeichnet, welches üblicherweise mittels Gelpermeationschromatographie (GPC) gegen Polystyrol als Standard bestimmt wird.
Als "Topfzeit" wird die maximale Zeitspanne ab dem Mischen der Komponenten und der Applikation einer Epoxidharz-Zusammensetzung bezeichnet, in der die vermischte Zusammensetzung in einem ausreichend fliessfähigen Zustand ist und die Substratoberflächen gut benetzen kann.
Als "Gelierzeit" wird die Zeitspanne ab dem Mischen der Komponenten einer Epoxidharz-Zusammensetzung bis zu deren Gelieren bezeichnet.
Als "Raumtemperatur" wird eine Temperatur von 23 °C bezeichnet.
Alle im Dokument erwähnten Industriestandards und Normen beziehen sich auf die zum Zeitpunkt der Einreichung der Erstanmeldung gültigen Fassungen, sofern nicht anders angegeben.
Gewichtsprozente (Gewichts-%) bezeichnen Massenanteile eines Bestandteils einer Zusammensetzung bezogen auf die gesamte Zusammensetzung, falls nichts anderes angegeben ist. Die Begriffe "Masse" und "Gewicht" werden im vorliegenden Dokument synonym benutzt.

Bevorzugt steht R¹ für H. Solche Mannichbasen sind abgeleitet von Formaldehyd oder einem Oligomeren oder Polymeren von Formaldehyd. Sie sind besonders gut herstellbar und ermöglichen besonders niedrigviskose Epoxidharz-Produkte mit besonders schneller Aushärtung. Zudem ist Formaldehyd aus biobasierter Quelle einfach verfügbar.

Bevorzugt steht A für 1,2-Ethylen, 1,2-Propylen, 1,3-Propylen, 1,3-Butylen, 1,4-Butylen, 2,2-Dimethyl-1,3-propylen, 1,3-Pentylen, 1,5-Pentylen, 2-Methyl-1,5-pentylen, 1,6-Hexylen, 1,7-Heptylen, 1,8-Octylen, 1,9-Nonylen, 2,2(4),4-Trimethyl-1,6-hexylen oder 1,10-Decylen.

Besonders bevorzugt steht A für einen linearen Alkylenrest mit 2 bis 6 C-Atomen, also für 1,2-Ethylen, 1,3-Propylen, 1,4-Butylen, 1,5-Pentylen oder 1,6-Hexylen, insbesondere für 1,2-Ethylen. Diese Mannichbasen sind besonders einfach mit hohem RCI erhältlich und ermöglichen besonders niedrigviskose Epoxidharz-Produkte mit besonders schneller Aushärtung.

Am meisten bevorzugt steht A für 1,2-Ethylen. Diese Mannichbasen verfügen unabhängig von der Aminquelle über einen besonders hohen RCI und ermöglichen ganz besonders niedrigviskose Epoxidharz-Produkte mit ganz besonders schneller Aushärtung.

In einer bevorzugten Ausführungsform der Erfindung steht X für einen linearen Kohlenwasserstoffrest mit 15 C-Atomen, der Rest Y para zu X steht für einen Rest der Formel (II), die weiteren Reste Y stehen für H und die Mannichbase weist somit die Formel (la) auf, 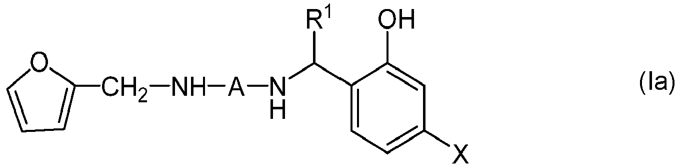 wobei A und R¹ die bereits genannten Bedeutungen aufweisen.

Die Mannichbase der Formel (la) ist abgeleitet von Cardanol, einem aus dem Öl der Cashewnussschale gewonnenen Alkenylphenol-Gemisch, und weisen einen ganz besonders hohen RCI auf. Sie wird auch als Phenalkamin bezeichnet. Dabei steht X für einen linearen C₁₅H₃₁- oder C₁₅H₂₉- oder C₁₅H₂₇- oder C₁₅H₂₅-Kohlenwasserstoffrest, insbesondere für die Reste der Formeln 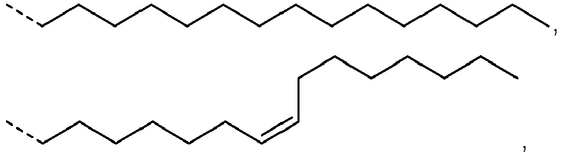 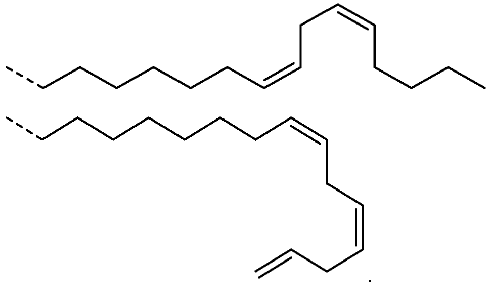 oder

Insbesondere stehen dabei A für 1,2-Ethylen und R¹ für H und die Mannichbase weist somit die Formel 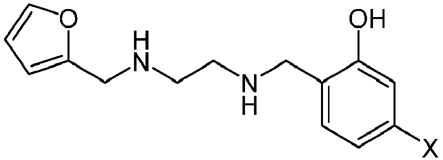 auf. Eine solche Mannichbase ist besonders nachhaltig und ermöglicht besonders niedrigviskose Epoxidharz-Produkte mit besonders schneller Aushärtung. Sie weist insbesondere einen RCI im Bereich von 0.9 bis 1 auf.

Eine solche Mannichbase ist insbesondere ein Reaktionsprodukt aus der Kondensation von mindestens einer Phenolverbindung der Formel (III) mit mindestens einem Amin der Formel (IV) und mindestens einem Aldehyd der Formel (V), 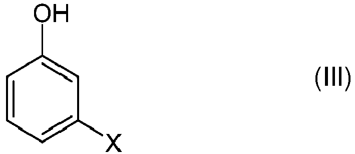 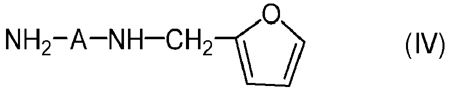 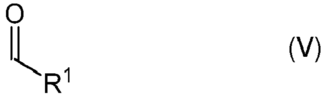 wobei X für einen linearen Kohlenwasserstoffrest mit 15 C-Atomen steht und A und R¹ die bereits genannten Bedeutungen aufweisen.

Bevorzugt werden die Phenolverbindung der Formel (III) und das Amin der Formel (IV) vorgelegt und der Aldehyd der Formel (V) langsam zugegeben, wobei die Temperatur der Reaktionsmischung bevorzugt im Bereich von 50 bis 150 °C gehalten wird. Anschliessend werden das freigesetzte Wasser und ein gegebenenfalls vorhandenens Lösemittel abdestilliert.
Pro mol Phenolverbindung der Formel (III) wird bevorzugt mindestens ein mol Amin der Formel (IV) und mindestens ein mol Aldehyd der Formel (V) eingesetzt. Besonders bevorzugt werden pro mol Phenolverbindung der Formel (III) 1 bis 10 mol, insbesondere 1 bis 5 mol, Amin der Formel (IV) und ca. 1 mol Aldehyd der Formel (V) eingesetzt. Dabei entsteht ein Reaktionsprodukt mit einem hohen Gehalt an Mannichbase der Formel (Ia) und gegebenenfalls überschüssigem Amin der Formel (IV). Ein solches Reaktionsprodukt ist besonders niedrigviskos.

Die Phenolverbindung der Formel (III) ist insbesondere Cardanol, ein aus dem Öl der Cashewnussschale gewonnenes Alkenylphenol-Gemisch, bei welchem X die bereits genannten Bedeutungen aufweist. Geeignet sind technische oder insbesondere destillierte und gegebenenfalls weiter gereinigte Qualitäten von Cardanol. Besonders geeignet sind kommerziell erhältliche Typen, wie beispielsweise die Cardolite Typen NX-2021, NX-2022, NX-2023, Ultra LITE 2023, NX-2024, NX-2025 oder NX-2026 (alle von Cardolite).

In einer weiteren bevorzgten Ausführungsform der Erfindung steht X für H, ein Rest Y ortho zur Phenolgruppe steht für Methoxy, die beiden weiteren Reste Y stehen jeweils für einen Rest der Formel (II) und die Mannichbase weist somit die Formel (Ib) auf, 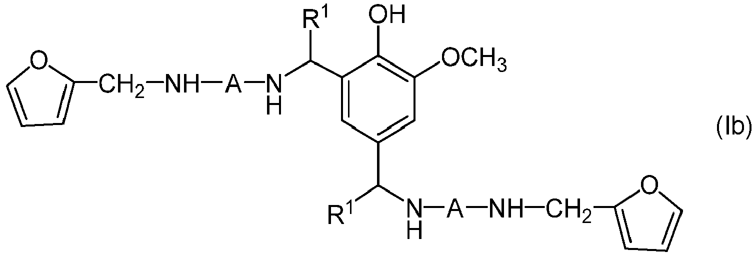 wobei R¹ und A die bereits beschriebenen Bedeutungen aufweisen.
Eine solche Mannichbase weist einen besonders hohen RCI auf und ermöglicht niedrigviskose Epoxidharz-Produkte mit besonders schneller Aushärtung. Bevorzugt liegt der RCI im Bereich von 0.8 bis 1.

Die Mannichbase der Formel (Ib) sind abgeleitet von Guajacol (o-Methoxyphenol) als Phenolverbindung der Formel (III). Sie sind insbesondere ein Reaktionsprodukt aus der Kondensation von Guajacol mit mindestens einem Amin der Formel (IV) und mindestens einem Aldehyd der Formel (V).
Bevorzugt wird biobasiertes Guajacol eingesetzt.

Bevorzugt erfolgt die Herstellung wie bereits für die Umsetzung mit Cardanol beschrieben, wobei pro mol Guajacol bevorzugt mindestens zwei mol Amin der Formel (IV) und mindestens zwei mol Aldehyd der Formel (V) eingesetzt werden. Besonders bevorzugt werden pro mol Guajacol 2 bis 10 mol, insbesondere 2 bis 5 mol, Amin der Formel (IV) und ca. 2 mol Aldehyd der Formel (V) eingesetzt. Dabei entsteht ein Reaktionsprodukt mit einem hohen Gehalt an Mannichbase der Formel (Ib) und gegebenenfalls überschüssigem Amin der Formel (IV).

In einer weiteren bevorzugten Ausführungsform der Erfindung steht in der Mannichbase der Formel (I) X für H, alle Reste Y stehen jeweils für einen Rest der Formel (II) und die Mannichbase weist somit die Formel (Ic) auf, 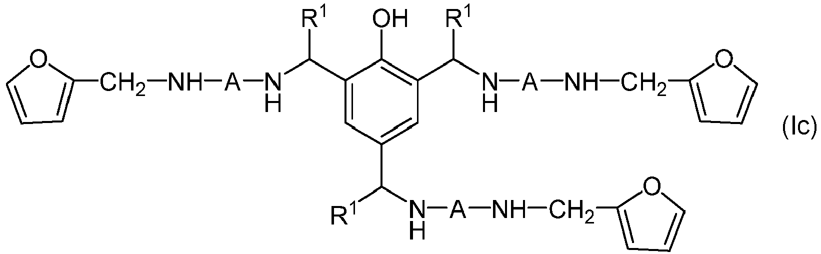 wobei R¹ und A die bereits genannten Bedeutungen aufweisen.
Eine Mannichbase der Formel (Ic) ermöglicht besonders hohe Endhärten.

Bevorzugt stehen R¹ dabei für H und A für 1,2-Ethylen und die Mannichbase der Formel (Ic) ist somit 2,4,6-Tris((2-furfurylaminoethyl)aminomethyl)phenol. Sie ermöglicht Epoxidharz-Produkte mit besonders schneller Aushärtung bei moderater Viskosität. Der RCI von 2,4,6-Tris((2-furfurylaminoethyl)aminomethyl)phenol liegt bevorzugt im Bereich von 0.5 bis 1, insbesondere 0.5 bis 0.6.

Eine Mannichbase der Formel (Ic) ist abgeleitet von Phenol als Phenolverbindung der Formel (III). Sie ist insbesondere ein Reaktionsprodukt aus der Kondensation von Phenol mit mindestens einem Amin der Formel (IV) und mindestens einem Aldehyd der Formel (V).
Bevorzugt erfolgt die Herstellung wie bereits für die Umsetzung mit Cardanol beschrieben, wobei pro mol Phenol bevorzugt mindestens drei mol Amin der Formel (IV) und mindestens drei mol Aldehyd der Formel (V) eingesetzt werden.

Besonders bevorzugt werden pro mol Phenol 3 bis 10 mol, insbesondere 3 bis 5 mol, Amin der Formel (IV) und ca. 3 mol Aldehyd der Formel (V) eingesetzt. Dabei entsteht ein Reaktionsprodukt mit einem hohen Gehalt an Mannichbase der Formel (Ic) und gegebenenfalls überschüssigem Amin der Formel (IV).

Als Amin der Formel (IV) für die Herstellung einer Mannichbase der Formel (I) geeignet sind Reaktionsprodukte aus der reduktiven Alkylierung von primären Diaminen der Formel H₂N-A-NH₂ mit Furfural und Wasserstoff. Solche Reaktionsprodukte enthalten typischerweise Nebenprodukte, insbesondere Anteile mit hydriertem Furanring und/oder dialkylierte Anteile, insbesondere wie in den folgenden Formeln dargestellt, 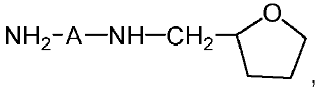 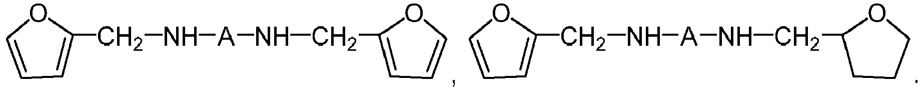 Entsprechend kann die Mannichbase der Formel (I), bzw. der Formel (Ia), (Ib) oder (Ic), Anteile von am Rest der Formel (II) hydriertem Furanring enthalten.

Besonders bevorzugt als Amin der Formel (IV) ist N-Furfuryl-1,2-ethandiamin, insbesondere mit einem RCI im Bereich von 0.7 bis 1.

Als Aldehyd der Formel (V) für die Herstellung einer Mannichbase der Formel (I) geeignet ist insbesondere Formaldehyd, Acetaldehyd, Propanal oder Benzaldehyd, gegebenenfalls in Form eines Oligomers oder Polymers. Besonders geeignet ist Formaldehyd, gegebenenfalls in Form von 1,3,5-Trioxan oder Paraformaldehyd. Bevorzugt ist Formaldehyd aus einer biobasierten Quelle.

In einer weiteren bevorzugten Ausführungsform der Erfindung stehen in der Mannichbase der Formel (I) X für H, ein oder zwei Reste Y für einen Rest der Formel (II) mit R¹ = H und die übrigen Reste Y jeweils für Dimethylaminomethyl. Eine solche Mannichbase der Formel (I) wird insbesondere erhalten aus der unvollständigen Umaminierung von 2,4,6-Tris(dimethylaminomethyl)phenol mit mindestens einem Amin der Formel (IV) unter Freisetzung von Dimethylamin.

In einer bevorzugten Ausführungsform der Erfindung ist eine Mannichbase der Formel (Ic) mit R¹ = H oder eine Mannichbase der Formel (I), bei welcher X für H, ein oder zwei Reste Y für einen Rest der Formel (II) mit R¹ = H und die übrigen Reste Y jeweils für Dimethylaminomethyl stehen, ein Reaktionsprodukt aus der Umaminierung von 2,4,6-Tris(dimethylaminomethyl)phenol mit mindestens einem Amin der Formel (IV) 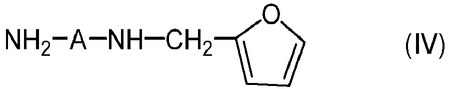 unter Freisetzung und Entfernung von Dimethylamin.

Bevorzugt wird dabei 2,4,6-Tris(dimethylaminomethyl)phenol mit dem Amin der Formel (IV) versetzt und bei einer Temperatur im Bereich von 80 bis 160 °C unter destillativer Entfernung von Dimethylamin erhitzt.
Bevorzugt werden pro mol 2,4,6-Tris(dimethylaminomethyl)phenol 1 bis 10 mol Amin der Formel (IV) eingesetzt.
Besonders bevorzugt werden pro mol 2,4,6-Tris(dimethylaminomethyl)phenol 3 bis 10 mol, insbesondere 3 bis 5 mol, Amin der Formel (IV) eingesetzt. Dabei entsteht hauptsächlich die Mannichbase der Formel (Ic).
Für den Fall, dass für die Umaminierung mehr als 3 mol Amin der Formel (IV) eingesetzt werden, enthält das Reaktionsprodukt gegebenenfalls überschüssiges Amin der Formel (IV). Ein solches Reaktionsprodukt ist besonders niedrigviskos und kann als solches für die Aushärtung von Epoxidharzen verwendet werden. Es kann aber auch gereinigt werden, indem überschüssiges Amin der Formel (IV) destillativ entfernt wird.
Für den Fall, dass die Umaminierung nur unvollständig durchgeführt wurde, beispielsweise indem pro mol 2,4,6-Tris(dimethylaminomethyl)phenol weniger als 3 mol Amin der Formel (IV) eingesetzt wurden, oder indem die Reaktion nicht vollständig durchgeführt wurde, entstehen Mannichbasen der Formel (I), bei welchen ein oder zwei Reste Y für Dimethylaminomethyl stehen.

Das für die Umaminierung eingesetzte 2,4,6-Tris(dimethylaminomethyl)phenol wird seinerseits insbesondere erhalten aus der Kondensation von Phenol mit Dimethylamin und Formaldehyd. Diese Umsetzung ist besonders einfach durchführbar und ermöglicht ein Produkt von besonders hoher Reinheit. 2,4,6-Tris-(dimethylaminomethyl)phenol ist kommerziell erhältlich, beispielsweise als Ancamine^{®} K54 (von Evonik) oder als Accelerator 960-1 (von Huntsman).

Als Amin der Formel (IV) geeignet sind die bereits beschriebenen Reaktionsprodukte aus der reduktiven Alkylierung von primären Diaminen der Formel H₂N-A-NH₂ mit Furfural und Wasserstoff. Entsprechend kann auch eine solche Mannichbase Anteile von am Rest der Formel (II) hydriertem Furanring enthalten.

In einer bevorzugten Ausführungsform wird bei der Umaminierung von 2,4,6-Tris(dimethylaminomethyl)phenol mit mindestens einem Amin der Formel (IV) zusätzlich ein Polyetherdiamin, insbesondere ein Polyoxypropylendiamin mit einem mittleren Molekulargewicht Mₙ im Bereich von 200 bis 500 g/mol, mitverwendet.
Bevorzugt werden dabei pro mol 2,4,6-Tris(dimethylaminomethyl)phenol ungefähr zwei mol Amin der Formel (IV) und 0.3 bis 0.7 mol Polyetherdiamin eingesetzt. Dabei wird insbesondere ein Reaktionsprodukt erhalten, welches zusätzlich zu mindestens einer Mannichbase der Formel (I) weitere Mannichbasen enthält, insbesondere auch Mannichbasen der Formel (VI),

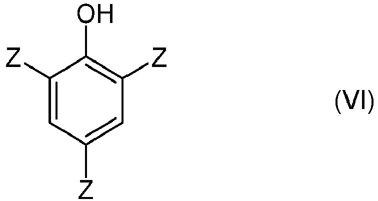

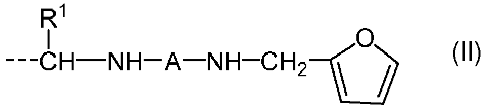

---CH₂-NH-B-NH-M (VII)

wobei
zwei der Reste Z für einen Rest der Formel (II) mit R¹ = H und der dritte Rest Z für einen Rest der Formel (VII) stehen,
B für einen zweiwertigen Polyetherrest steht, insbesondere für Polyoxypropylen mit einem mittleren Molekulargewicht Mₙ im Bereich von 170 bis 470 g/mol,
M für H oder einen Rest der Formel (VIIIa) oder (VIIIb) steht, 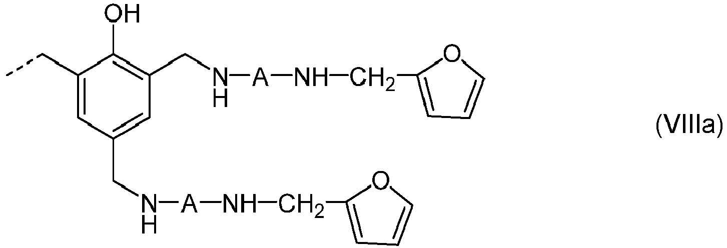 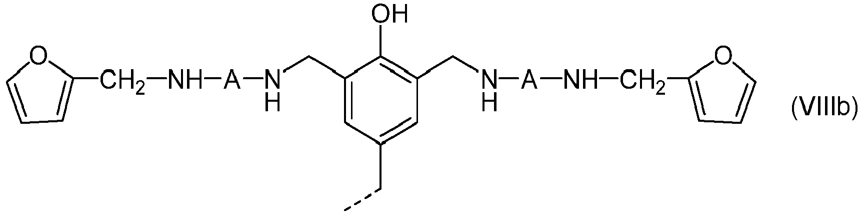
und A die bereits beschriebenen Bedeutungen aufweist.
Eine solches Gemisch aus Mannichbasen ist besonders niedrigviskos und polymer, was die Handhabung erleichtert und die Flüchtigkeit senkt.

Ein weiterer Gegenstand der Erfindung ist ein Härter für Epoxidharze enthaltend mindestens eine Mannichbase der Formel (I), wie vorgängig beschrieben. Bevorzugt enthält der Härter 1 bis 95 Gewichts-%, mehr bevorzugt 2 bis 80 Gewichts-%, besonders bevorzugt 3 bis 60 Gewichts-%, insbesondere 3 bis 30 Gewichts-%, Mannichbasen der Formel (I).

Der Härter enthält bevorzugt mindestens einen weiteren Bestandteil ausgewählt aus weiteren Aminen, welche nicht der Formel (I) entsprechen, Beschleunigern und Verdünnern, insbesondere mindestens ein weiteres Amin, welches nicht der Formel (I) entspricht.

Als weiteres Amin, welches nicht der Formel (I) entspricht, besonders bevorzugt ist ein Amin der Formel (IV), wie vorgängig beschrieben, insbesondere N-Furfuryl-1,2-ethandiamin. Ein solcher Härter hat einen hohen RCI und ermöglicht besonders niedrigviskose Epoxidharz-Produkte mit schneller und störungsfreier Aushärtung.
Bevorzugt enthält der Härter Amine der Formel (IV) und Mannichbasen der Formel (I) in einem Gewichtsverhältnis im Bereich von 5/95 bis 99/1, bevorzugt 10/90 bis 98/2, besonders bevorzugt 25/75 bis 95/5, insbesondere 50/50 bis 95/5.

Als weiteres Amin, welches nicht der Formel (I) entspricht, besonders bevorzugt ist weiterhin mindestens ein Amin mit einem hohen RCI, insbesondere ausgewählt aus 2,5-Bis(aminomethyl)furan, 2,5-Bis(aminomethyl)tetrahydrofuran, Bis(5-aminomethylfuran-2-yl)methan, Bis(5-aminomethyltetrahydrofuran-2-yl)methan, 2,2-Bis(5-aminomethylfuran-2-yl)propan und 2,2-Bis(5-aminomethyltetrahydrofuran-2-yl)propan.

Als weiteres Amin, welches nicht der Formel (I) entspricht, geeignet sind weiterhin insbesondere Amine mit aliphatischen Aminogruppen und mindestens drei Aminwasserstoffen, insbesondere N-Benzyl-1,2-ethandiamin, N-Benzyl-1,2-propandiamin, N-Benzyl-1,3-bis(aminomethyl)benzol, N-(2-Ethylhexyl)-1,3-bis(aminomethyl)benzol, 2,2-Dimethyl-1,3-propandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2(4),4-Trimethyl-1,6-hexandiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3-oder 1,4-Diaminocyclohexan, 1,3-Bis(aminomethyl)cyclohexan, 1,4-Bis(aminomethyl)cyclohexan, Bis(4-aminocyclohexyl)methan, Bis(4-amino-3-methylcyclohexyl)-methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (IPDA), 2(4)-Methyl-1,3-diaminocyclohexan, 2,5(2,6)-Bis(aminomethyl)bicyclo[2.2.1]heptan (NBDA), 3(4),8(9)-Bis(aminomethyl)tricyclo[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol (MXDA), 1,4-Bis(aminomethyl)benzol, Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder höhere Oligomere dieser Diamine, Bis(3-aminopropyl)polytetrahydrofurane oder andere Polytetrahydrofurandiamine, Polyoxyalkylendi- oder -triamine, insbesondere Polyoxypropylendiamine oder Polyoxypropylentriamine wie Jeffamine^{®} D-230, Jeffamine^{®} D-400 oder Jeffamine^{®} T-403 (alle von Huntsman), Diethylentriamin (DETA), Triethylentetramin (TETA), Tetraethylenpentamin (TEPA), Pentaethylenhexamin (PEHA), Dipropylentriamin (DPTA), N-(2-Aminoethyl)-1,3-propandiamin (N3-Amin), N,N'-Bis(3-aminopropyl)-ethylendiamin (N4-Amin), N,N'-Bis(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin, N,N'-Bis(3-amino-1-ethylpropyl)-2-methyl-1,5-pentandiamin, 3-(2-Aminoethyl)aminopropylamin, Bis(hexamethylen)triamin (BHMT), N-Aminoethylpiperazin, 3-(3-(Dimethylamino)propyl-amino)propylamin (DMAPAPA), aminfunktionelle Addukte der genannten Amine mit Epoxiden, Phenalkamine, welche Umsetzungsprodukte von Cardanol mit Aldehyden, insbesondere Formaldehyd, und Polyaminen darstellen, oder eine Mischung aus zwei oder mehr dieser Amine.

Davon bevorzugt sind N-Benzyl-1,2-ethandiamin, MPMD, TMD, 1,2-Diaminocyclohexan, 1,3-Bis(aminomethyl)cyclohexan, 1,4-Bis(aminomethyl)cyclohexan, Bis(4-aminocyclohexyl)methan, IPDA, 2(4)-Methyl-1,3-diaminocyclohexan, MXDA, DETA, TETA, TEPA, N3-Amin, N4-Amin, DPTA, BHMT, Polyoxypropylendiamine mit einem mittlerem Molekulargewicht Mₙ im Bereich von 200 bis 500 g/mol oder Polyoxypropylentriamine mit einem mittlerem Molekulargewicht Mₙ im Bereich von 300 bis 500 g/mol.
Besonders bevorzugt ist 1,3-Bis(aminomethyl)cyclohexan. Damit wird eine besonders schnelle Aushärtung ermöglicht.
Besonders bevorzugt ist weiterhin IPDA. Damit werden besonders hohe Glasübergangstemperaturen erreicht, was eine besonders gute Robustheit gegenüber hohen Gebrauchstemperaturen ermöglicht. Besonders bevorzugt wird IPDA mit einem hohen RCI aus biobasiertem Aceton eingesetzt.

Besonders bevorzugt ist weiterhin MXDA. Damit werden hohe Aushärtegeschwindigkeiten und besonders hohe Festigkeiten erreicht.
Besonders bevorzugt ist weiterhin N-Benzyl-1,2-ethandiamin. Damit werden Epoxidharz-Produkte mit besonders schönen Oberflächen erreicht.

Geeignete Beschleuniger sind insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; Nitrate wie insbesondere Calciumnitrat; tertiäre Amine wie insbesondere 1,4-Diazabicyclo[2.2.2]octan, Benzyldimethylamin, α-Methylbenzyldimethylamin, Triethanolamin, Dimethylaminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo[5.4.0]-undec-7-en, Guanidine wie insbesondere 1,1,3,3-Tetramethylguanidin, Phenole, insbesondere Bisphenole, Phenol-Harze oder weitere Mannich-Basen wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin, Phosphite wie insbesondere Di- oder Triphenylphosphite, oder Mercaptogruppen aufweisende Verbindungen.
Bevorzugt sind Säuren, Nitrate, tertiäre Amine oder weitere Mannich-Basen, insbesondere Salicylsäure, Calciumnitrat oder 2,4,6-Tris(dimethylaminomethyl)-phenol, oder eine Kombination dieser Beschleuniger.

Geeignete Verdünner sind insbesondere n-Propanol, Isopropanol, n-Butanol, Isobutanol, tert.Butanol, 1-Pentanol, 2-Pentanol, 3-Pentanol, 2-Methyl-1-butanol, 3-Methyl-1-butanol, 3-Methyl-2-butanol n-Hexanol, 2-Ethylhexanol, Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykolmonomethylether, Diethylenglykolmonoethylether, Diethylenglykolmono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, 2,2,4-Trimethyl-1,3-pentandiolmonoisobutyrat, Diphenylmethan, Diisopropylnaphthalin, Erdölfraktionen wie zum Beispiel Solvesso^{®}-Typen (von Exxon), Alkylphenole wie tert.Butylphenol, Nonylphenol, Dodecylphenol, Cardanol, styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- oder Sulfonsäureester oder Sulfonamide.

Davon bevorzugt sind Verdünner mit einem Siedepunkt von mehr als 200 °C, insbesondere Benzylalkohol, styrolisiertem Phenol, ethoxyliertes Phenol, phenolgruppenhaltige aromatische Kohlenwasserstoffharze wie insbesondere die Novares^{®}-Typen LS 500, LX 200, LA 300 oder LA 700 (von Rütgers), Diisopropylnaphthalin oder Cardanol, insbesondere Benzylalkohol. Phenolgruppen-haltige Verdünner zeigen auch eine Wirkung als Beschleuniger. Davon bevorzugt sind weiterhin aromatische Verdünner mit einer besonders hohen verdünnenden Wirkung, insbesondere Xylol.
Davon besonders bevorzugt sind Verdünner mit einem RCI von 1, insbesondere Cardanol. Diese ermöglichen einen besonders nachhaltigen Härter.

Bevorzugt enthält der Härter nur einen geringen Gehalt an Verdünnern, insbesondere 0 bis 50 Gewichts-%, bevorzugt 0 bis 30 Gewichts-%, Verdünner bezogen auf den gesamten Härter.

Der Härter kann wasserbasiert sein und Wasser im Bereich von 15 bis 90 Gewichts-%, bevorzugt 20 bis 80 Gewichts-%, enthalten.

Der Härter ist bevorzugt nicht wasserbasiert. Er enthält bevorzugt weniger als 15 Gewichts-%, insbesondere weniger als 10 Gewichts-%, Wasser, bezogen auf den gesamten Härter. Ein solcher Härter ist besonders geeignet für nicht-wässrige Epoxidharze. Er ermöglicht Epoxidharz-Produkte mit besonders hoher Robustheit gegenüber Feuchtigkeit.

Der Härter kann weitere Bestandteile enthalten, insbesondere:
- aminfunktionelle Addukte mit Epoxiden
- Monoamine wie insbesondere Benzylamin oder Furfurylamin,
- Amine mit zwei Aminwasserstoffen, insbesondere N,N'-Difurfuryl-1,2-ethandiamin, N,N'-Dibenzyl-1,2-ethandiamin oder 3-Dimethylaminopropylamin (DMAPA),
- Polyamidoamine, insbesondere Umsetzungsprodukte aus einer ein- oder mehrwertigen Carbonsäure oder deren Ester oder Anhydrid, insbesondere einer Dimerfettsäure, mit einem im stöchiometrischen Überschuss eingesetzten Polyamin, insbesondere DETA oder TETA,
- aromatische Polyamine wie insbesondere 4,4'-, 2,4' und/oder 2,2'-Diaminodiphenylmethan, 2,4(6)-Toluoldiamin, 3,5-Dimethylthio-2,4(6)-toluoldiamin oder 3,5-Diethyl-2,4(6)-toluylendiamin,
- Mercaptogruppen aufweisende Verbindungen, insbesondere flüssige Mercaptan-terminierte Polysulfidpolymere, Mercaptan-terminierte Polyoxyalkylenether, Mercaptan-terminierte Polyoxyalkylen-Derivate, Polyester von Thiocarbonsäuren, 2,4,6-Trimercapto-1,3,5-triazin, Triethylenglykoldimercaptan oder Ethandithiol,
- oberflächenaktive Additive, insbesondere Entschäumer, Entlüfter, Netzmittel, Dispergiermittel oder Verlaufsmittel, oder
- Stabilisatoren, insbesondere Stabilisatoren gegen Oxidation, Wärme, Licht oder UV-Strahlung.

Ein weiterer Gegenstand der Erfindung ist eine Epoxidharz-Zusammensetzung umfassend
- eine Harz-Komponente umfassend mindestens ein Epoxidharz und
- eine Härter-Komponente umfassend mindestens eine Mannichbase der Formel (I), wie vorgängig beschrieben.

Ein geeignetes Epoxidharz wird auf bekannte Art und Weise erhalten, insbesondere aus der Reaktion von Epichlorhydrin mit Polyolen, Polyphenolen oder Aminen.

Geeignete Epoxidharze sind insbesondere aromatische Epoxidharze, insbesondere die Glycidylether von:
- Bisphenol A, Bisphenol F oder Bisphenol A/F, wobei A für Aceton und F für Formaldehyd steht, welche als Edukte zur Herstellung dieser Bisphenole dienten. Im Fall von Bisphenol F können auch Stellungsisomere vorhanden sein, insbesondere abgeleitet von 2,4'- oder 2,2'-Hydroxyphenylmethan.
- Dihydroxybenzol-Derivaten wie Resorcin, Hydrochinon oder Brenzcatechin;
- weiteren Bisphenolen oder Polyphenolen wie Bis(4-hydroxy-3-methylphenyl)-methan, 2,2-Bis(4-hydroxy-3-methylphenyl)propan (Bisphenol-C), Bis(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)-heptan, 2,4-Bis(4-hydroxyphenyl)-2-methylbutan, 2,4-Bis(3,5-dimethyl-4-hydroxyphenyl)-2-methylbutan, 1,1-Bis(4-hydroxyphenyl)cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]-benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxynaphth-1-yl)methan, Bis(4-hydroxynaphth-1-yl)methan, 1,5-Dihydroxynaphthalin, Tris(4-hydroxyphenyl)methan, 1,1,2,2-Tetrakis(4-hydroxyphenyl)ethan, Bis(4-hydroxyphenyl)ether oder Bis(4-hydroxyphenyl)sulfon;
- Novolaken, welche insbesondere Kondensationsprodukte von Phenol oder Kresolen mit Formaldehyd bzw. Paraformaldehyd oder Acetaldehyd oder Crotonaldehyd oder Isobutyraldehyd oder 2-Ethylhexanal oder Benzaldehyd oder Furfural sind;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylenbis(1-methylethyliden)]bisanilin (Bisanilin-P) oder 4,4'-[1,3-Phenylen-bis(1 - methylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, zyklischen oder offenkettigen di-, tri- oder tetrafunktionellen C₂- bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromoneopentylglykol, Rizinusöl, Trimethylolpropan, Trimethylolethan, Pentaerythrol, Sorbit oder Glycerin, oder alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat oder Triglycidylisocyanurat, oder Umsetzungsprodukte von Epichlorhydrin mit Hydantoin.

Weitere geeignete Epoxidharze sind Epoxidharze mit einem hohen RCI, insbesondere solche aus der Umsetzung von biobasierten hydroxyfunktionellen Rohstoffen mit biobasiertem Epichlorhydrin. Besonders bevorzugt sind Vanillinbasierte Epoxidharze wie insbesondere Vanillinalkohol-Diglycidylether oder die Glycidylether von Bisvanillin-Derivaten, sowie Glycerol-basierte Epoxidharze wie insbesondere die Glycidylether von Glycerol oder Polyglycerol.

Bevorzugt ist das Epoxidharz ein Flüssigharz oder eine Mischung enthaltend zwei oder mehr Epoxid-Flüssigharze.
Als "Epoxid-Flüssigharz" wird ein technisches Polyepoxid mit einer Glasübergangstemperatur unterhalb von 25 °C bezeichnet.
Gegebenenfalls enthält die Harz-Komponente zusätzlich Anteile von Epoxid-Festharz.

Das Epoxidharz ist insbesondere ein Flüssigharz auf der Basis eines Bisphenols oder Novolaks, insbesondere mit einem mittleren Epoxid-Equivalentgewicht im Bereich von 156 bis 210 g/eq.

Besonders geeignet ist ein Bisphenol A-Diglycidylether und/oder Bisphenol F-Diglycidylether, wie sie kommerziell beispielsweise von Olin, Huntsman oder Momentive erhältlich sind. Diese Flüssigharze weisen eine für Epoxidharze niedrige Viskosität auf und ermöglichen eine schnelle Aushärtung und hohe Härten. Sie können Anteile von Bisphenol A-Festharz oder Novolak-Epoxidharzen enthalten.
Besonders bevorzugt ist ein Bisphenol A-Diglycidylether mit einem RCI von 0.3 aus der Umsetzung von Bisphenol A mit biobasiertem Epichlorhydrin.

Weiterhin besonders bevorzugt sind Phenol-Formaldehyd Novolak-Glycidylether, insbesondere mit einer mittleren Funktionalität im Bereich von 2.3 bis 4, bevorzugt 2.5 bis 3. Sie können Anteile von weiteren Epoxidharzen enthalten, insbesondere Bisphenol A-Diglycidylether oder Bisphenol F-Diglycidylether.

Weiterhin besonders bevorzugt sind Epoxidharze mit einem hohen RCI, insbesondere Vanillinalkohol-Diglycidylether oder die Glycidylether von Glycerol oder Polyglycerol.

Die Harz-Komponente kann einen Reaktivverdünner enthalten.
Als Reaktivverdünner bevorzugt sind Epoxidgruppen-haltige Reaktivverdünner, insbesondere Butandioldiglycidylether, Hexandioldiglycidylether, Trimethylolpropandi- oder triglycidylether, Phenylglycidylether, Kresylglycidylether, Guajacolglycidylether, 4-Methoxyphenylglycidylether, p-n-Butylphenylglycidylether, p-tert.Butylphenylglycidylether, 4-Nonylphenylglycidylether, 4-Dodecylphenylglycidylether, Cardanolglycidylether, Benzylglycidylether, Allylglycidylether, Butylglycidylether, Hexylglycidylether, 2-Ethylhexylglycidylether, oder Glycidylether von natürlichen Alkoholen wie insbesondere C₈- bis C₁₀- oder C₁₂- bis C₁₄- oder C₁₃-bis C₁₅-Alkylglycidylether.

Bevorzugt enthält die Epoxidharz-Zusammensetzung mindestens ein Epoxidharz mit einem RCI im Bereich von 0.2 bis 1, insbesondere 0.5 bis 1. Dies ermöglicht besonders nachhaltige Epoxidharz-Produkte.

Bevorzugt umfasst die Härter-Komponente der Epoxidharz-Zusammensetzung einen Härter enthaltend mindestens eine Mannichbase der Formel (I), wie vorgängig beschrieben.
Insbesondere umfasst die Härter-Komponente zusätzlich mindestens ein Amin der Formel (IV), wie vorgängig beschrieben.

Bevorzugt enthält die Epoxidharz-Zusammensetzung mindestens einen weiteren Bestandteil ausgewählt aus der Gruppe bestehend aus Verdünnern, Beschleunigern, Füllstoffen, Pigmenten und oberflächenaktiven Additiven.

Als Verdünner oder Beschleuniger geeignet sind insbesondere die bereits genannten.

Geeignete Füllstoffe sind insbesondere gemahlenes oder gefälltes Calciumcarbonat, welches gegebenenfalls mit Fettsäure, insbesondere Stearaten, beschichtet ist, Baryt (Schwerspat), Talk, Quarzmehl, Quarzsand, Siliciumcarbid, Eisenglimmer, Dolomit, Wollastonit, Kaolin, Mica (Kalium-Aluminium-Silikat), Molekularsiebe, Aluminiumoxid, Zinkoxid, Aluminium-dotiertes Zinkoxid, Aluminiumhydroxid, Magnesiumhydroxid, Kieselsäure, Zement, Gips, Flugasche, Russ, Graphit, Metall-Pulver wie Aluminium, Kupfer, Eisen, Zink, Silber oder Stahl, PVC-Pulver oder Hohlkugeln, sowie biobasierte Füllstoffe wie beispielsweise Ligninpulver oder gemahlene Nussschalen oder Obststeine. Davon bevorzugt sind Calciumcarbonat, Baryt, Quarzmehl, Talk, Aluminiumpulver, biobasierte Füllstoffe oder eine Kombination davon.

Geeignete Pigment sind insbesondere Titandioxide, Eisenoxide, Chrom(III)oxide, organische Pigmente, Russ oder Korrosionsschutzpigmente, insbesondere Phosphate, Orthophosphate oder Polyphosphate, welche als Gegenion insbesondere Chrom, Zink, Aluminium, Kalzium, Strontium oder eine Kombination dieser Metalle enthalten. Besonders geeignet sind Titandioxide.

Geeignete oberflächenaktive Additive sind insbesondere Entschäumer, Entlüfter, Netzmittel, Dispergiermittel, Verlaufsmittel und/oder dispergierte Paraffinwachse.

Gegebenenfalls enthält die Epoxidharz-Zusammensetzung weitere Hilfs- und Zusatzstoffe, insbesondere die Folgenden:
- Reaktivverdünner, insbesondere die bereits vorgängig erwähnten, oder epoxidiertes Sojaöl oder Leinöl, Acetoacetatgruppen aufweisende Verbindungen, insbesondere acetoacetylierte Polyole, Butyrolakton, Carbonate, Aldehyde, Isocyanate oder Reaktivgruppen aufweisende Silikone;
- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere chlorsulfonierte Polyethylene oder Fluor-haltige Polymere oder Sulfonamid-modifizierte Melamine;
- Fasern, insbesondere Glasfasern, Kohlefasern, Metallfasern, Keramikfasern, Kunststofffasern wie Polyamidfasern oder Polyethylenfasern oder biobasierte Fasern wie insbesondere Holzfasern, Zellulosefasern, Hanffasern, Flachs (Leinen), Jute oder Kokosfasern;
- Nanofüllstoffe, insbesondere Carbon Nanotubes;
- Rheologie-Modifizierer, insbesondere Verdicker oder Antiabsetzmittel;
- Haftverbesserer, insbesondere Organoalkoxysilane;
- flammhemmende Substanzen, insbesondere die bereits genannten Füllstoffe Aluminiumhydroxid oder Magnesiumhydroxid, Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinoldiphosphat-Oligomer, Tetraphenylresorcinoldiphosphit, Ethylendiamindiphosphat, Bisphenol-A-bis(diphenylphosphat), Tris(chloroethyl)phosphat, Tris(chloropropyl)-phosphat, Tris(dichloroisopropyl)phosphat, Tris[3-bromo-2,2-bis(bromomethyl)-propyl]phosphat, Tetrabromo-Bisphenol-A, Bis(2,3-dibromopropylether) von Bisphenol A, bromierte Epoxidharze, Ethylen-bis(tetrabromophthalimid), Ethylen-bis(dibromonorbornandicarboximid), 1,2-Bis(tribromophenoxy)ethan, Tris(2,3-dibromopropyl)isocyanurat, Tribromophenol, Hexabromocyclododecan, Bis(hexachlorocyclopentadieno)cyclooctan oder Chlorparaffine; oder
- Stabilisatoren gegen Oxidation, Wärme, Licht oder UV-Strahlung oder Biozide.

Bevorzugt enthält die Epoxidharz-Zusammensetzung nur einen geringen Gehalt an Verdünnern. Bevorzugt enthält sie weniger als 20 Gewichts-%, besonders bevorzugt weniger als 10 Gewichts-%, insbesondere weniger als 5 Gewichts-%, , am meisten bevorzugt weniger als 1 Gewichts-% Verdünner.

Die Epoxidharz-Zusammensetzung kann Wasser enthalten.
In einer Ausführungsform ist die Epoxidharz-Zusammensetzung wasserbasiert. Dabei ist das Epoxidharz bevorzugt in einer Menge von 50 bis 85 Gewichts-% in Wasser emulgiert und die Härter-Komponente enthält bevorzugt 20 bis 80 Gewichts-% Wasser.
Bevorzugt enthält die Epoxidharz-Zusammensetzung nur einen geringen Gehalt an Wasser, bevorzugt weniger als 5 Gewichts-%, insbesondere weniger als 1 Gewichts-%, Wasser. Eine solche, nicht-wasserbasierte Epoxidharz-Zusammensetzung ist besonder vielseitig verwendbar und besonders wasserbeständig.

Bevorzugt ist eine Epoxidharz-Zusammensetzung umfassend
- eine Harz-Komponente enthaltend mindestens ein Epoxidharz und gegebenenfalls weitere Bestandteile wie insbesondere Epoxidgruppen-haltige Reaktivverdünner, Verdünner, Füllstoffe, Pigmente und/oder oberflächenaktive Additive, und
- eine Härter-Komponente enthaltend mindestens eine Mannichbase der Formel (I) und gegebenenfalls weitere Bestandteile wie insbesondere weitere Amine, Beschleuniger und/oder Verdünner.

Die Harz- und die Härter-Komponente der Epoxidharz-Zusammensetzung werden in voneinander getrennten Gebinden gelagert.

Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern.

Die Harz- und die Härter-Komponente werden kurz vor oder während der Applikation vermischt. Das Mischungsverhältnis wird bevorzugt so gewählt, dass das molare Verhältnis der gegenüber Epoxidgruppen reaktiven Gruppen zu den Epoxidgruppen im Bereich von 0.5 bis 1.5, insbesondere 0.7 bis 1.2, liegt. In Gewichtsteilen liegt das Mischungsverhältnis zwischen der Harz- und der Härter-Komponente typischerweise im Bereich von 1:2 bis 20:1.
Das Mischen der Komponenten erfolgt kontinuierlich oder batchweise mittels eines geeigneten Verfahrens, wobei darauf zu achten ist, dass zwischen dem Mischen der Komponenten und der Applikation nicht zu viel Zeit vergeht und die Applikation innerhalb der Topfzeit erfolgt. Das Mischen und die Applikation können bei Umgebungstemperatur erfolgen, welche typischerweise im Bereich von etwa 5 bis 40°C, bevorzugt bei etwa 10 bis 35°C, liegt, oder bei erhöhter Temperatur, insbesondere im Bereich von 40 bis 150 °C, bevorzugt 50 bis 120 °C.

Mit dem Mischen der Komponenten beginnt die Aushärtung der Epoxidharz-Zusammensetzung durch chemische Reaktion. Primäre und sekundäre Aminogruppen und gegebenenfalls vorhandene weitere gegenüber Epoxidgruppen reaktive Gruppen reagieren mit den Epoxidgruppen unter deren Ringöffnung. Weiterhin katalysiert die Mannichbase der Formel (I) die Homopolymerisation der Epoxidgruppen. Als Ergebnis hauptsächlich dieser Reaktionen polymerisiert die Zusammensetzung und härtet dadurch aus.

Die Aushärtung erstreckt sich typischerweise über einige Stunden bis Tage. Die Dauer hängt unter anderem von der Temperatur, der Reaktivität der Bestandteile, deren Stöchiometrie und der Gegenwart bzw. Menge von Beschleunigern ab.

Im frisch vermischten Zustand hat die Epoxidharz-Zusammensetzung eine niedrige Viskosität. Bevorzugt liegt die Viskosität 5 Minuten nach dem Mischen der Harz- und der Härter-Komponente bei 20 °C im Bereich von 0.1 bis 20 Pa·s, bevorzugt 0.2 bis 10 Pa·s, besonders bevorzugt 0.25 bis 5 Pa·s, gemessen mittels Kegel-Platten Viskosimeter bei einer Schergeschwindigkeit von 10 s⁻¹.

Die Applikation der Epoxidharz-Zusammensetzung erfolgt auf mindestens ein Substrat und/oder in mindestens eine Giessform.

Als Substrat geeignet sind insbesondere:
- Glas, Glaskeramik, Beton, Mörtel, Zementestrich, Faserzement, Backstein, Ziegel, Gips oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Eisen, Stahl, Kupfer, weitere Buntmetalle, inklusive oberflächenveredelte Metalle oder Legierungen wie verzinkte oder verchromte Metalle;
- Asphalt oder Bitumen;
- Leder, Textilien, Papier, Holz, mit Harzen, beispielsweise Phenol-, Melamin- oder Epoxidharzen, gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- Faserverstärkte Kunststoffe, wie Kohlefaser-verstärkte Kunststoffe (CFK), Glasfaser-verstärkte Kunststoffe (GFK), Naturfaser-verstärkte Kunststoffe (NFK) und Sheet Moulding Compounds (SMC);
- Isolierschäume, insbesondere aus EPS, XPS, PUR, PIR, Steinwolle, Glaswolle oder geschäumtem Glas (Foamglas);
- beschichtete oder lackierte Substrate, insbesondere lackierte Fliesen, gestrichener Beton, pulverbeschichtete Metalle oder Legierungen oder lackierte Bleche;
- Beschichtungen, Farben oder Lacke, insbesondere beschichtete Böden, welche mit einer weiteren Bodenbelagsschicht überschichtet werden.
Die Substrate können bei Bedarf vor dem Applizieren vorbehandelt werden, insbesondere durch physikalische und/oder chemische Reinigungsverfahren oder das Aufbringen eines Aktivators oder eines Primers.

Die Substrate werden insbesondere beschichtet und/oder verklebt.

Als Giessform geeignet ist eine Vorrichtung, in welche die vermischte, flüssige Epoxidharz-Zusammensetzung gegossen und darin ausgehärtet werden und nach der Aushärtung daraus entformt bzw. entnommen werden kann, wobei die ausgehärtete Zusammensetzung einen Formkörper bildet.
Die Giessform besteht bevorzugt zumindest auf der Oberfläche aus einem Material, von welchem die ausgehärtete Epoxidharz-Zusammensetzung ohne Beschädigung wieder gelöst werden kann, insbesondere aus Metall, Keramik, Kunststoff oder Silikon, welche gegebenenfalls mit einer Antihaft-Beschichtung versehen sind, insbesondere aus Teflon, Silikon oder einem Wachs.

Ein weiterer Gegenstand der Erfindung ist eine ausgehärtete Zusammensetzung erhalten aus der beschriebenen Epoxidharz-Zusammensetzung nach dem Vermischen der Harz- und der Härter-Komponente.

Die Epoxidharz-Zusammensetzung wird bevorzugt verwendet als Beschichtung, Primer, Klebstoff, Dichtstoff, Vergussmasse, Giessharz, Imprägnierharz, oder als Formkörper oder Matrix für Verbundwerkstoffe (Composites) wie insbesondere CFK (enthaltend Carbonfasern), GFK (enthaltend Glasfasern), NFK (enthaltend Naturfasern) oder Holzverbundwerkstoffe.

Aus der Verwendung entsteht ein Artikel enthaltend die ausgehärtete Zusammensetzung aus der beschriebenen Epoxidharz-Zusammensetzung.

Der Artikel ist insbesondere eine Bodenbeschichtung, Wandbeschichtung, Bauteilbeschichtung, Rohrbeschichtung, Dachbeschichtung oder eine Korrosionsschutzbeschichtung, oder ein verklebter Artikel, oder ein Formkörper, insbesondere ein Composit-Material.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

"AHEW" steht für das Aminwasserstoff-Equivalentgewicht.

"EEW" steht für das Epoxid-Equivalentgewicht.

Als "Normklima" ("NK") wird eine Temperatur von 23±1 °C und eine relative Luftfeuchtigkeit von 50±5% bezeichnet.

Die verwendeten Chemikalien waren, sofern nicht anders bezeichnet, von Sigma-Aldrich Chemie GmbH.

### Beschreibung der Messmethoden:

Die **Viskosität** wurde auf einem thermostatisierten Kegel-Platten-Viskosimeter Rheotec RC30 (Kegeldurchmesser 50 mm, Kegelwinkel 1°, Kegelspitze-PlattenAbstand 0.05 mm, Scherrate 10 s⁻¹) gemessen. Viskositäten von weniger als 100 mPa·s wurden mit einer Scherrate von 100 s⁻¹ gemessen.

Die **Aminzahl** wurde durch Titration bestimmt (mit 0.1N HClO₄ in Essigsäure gegen Kristallviolett).

**Gaschromatogramme** (GC) wurden gemessen im Temperaturbereich von 60 bis 320 °C mit einer Aufheizrate von 15 °C/min und 10 min Verweilzeit bei 320 °C. Die Injektortemperatur betrug 250 °C. Es wurde eine Zebron ZB-5 Säule verwendet (L = 30 m, ID = 0.25 mm, dj = 0.5 µm) bei einem Gasfluss von 1.5 ml/Min. Die Detektion erfolgte mittels Flammenionisation (FID).

**Infrarotspektren** (FT-IR) wurden als unverdünnte Filme auf einem mit horizontaler ATR-Messeinheit mit Diamant-Kristall ausgestatteten FT-IR Gerät Nicolet iS5 von Thermo Scientific gemessen. Die Absorptionsbanden sind in Wellenzahlen (cm⁻¹) angegeben.

**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Echte und Pseudo-Kopplungsmuster wurden nicht unterschieden.

### Verwendete Substanzen und Abkürzungen:

- Ancamine^{®} K54:: 2,4,6-Tris(dimethylaminomethyl)phenol (von Evonik)
- Jeffamine^{®} D-230:: Polyoxypropylendiamin, mittleres Molekulargewicht 230 g/mol, AHEW 60 g/eq (von Huntsman)
- F-EDA:: N-Furfuryl-1,2-ethandiamin, AHEW 46.7 g/eq, RCI 0.71, hergestellt wie nachfolgend beschrieben
- B-EDA:: N-Benzyl-1,2-ethandiamin, AHEW 50.1 g/eq, hergestellt wie nachfolgend beschrieben
- Araldite^{®} GY 250:: Bisphenol A-Diglycidylether, EEW ca. 187 g/eq (von Huntsman)
- Araldite^{®} DY-E:: Monoglycidylether von C₁₂- bis C₁₄-Alkoholen, EEW ca. 290 g/eq (von Huntsman)

### Herstellung von Aminen:

### N-Furfuryl-1,2-ethandiamin (F-EDA):

In einem Rundkolben wurden 105.2 g (1.75 mol) 1,2-Ethandiamin unter Stickstoffatmosphäre bei Raumtemperatur vorgelegt. Unter gutem Rühren wurde eine Lösung aus 48.05 g g (0.5 mol) Furfural (Furan-2-carbaldehyd, RCI = 1) in 200 ml Isopropanol zugegeben und 1 Stunde bei 40 °C nachgerührt. Die Reaktionsmischung wurde mit weiteren 1'000 ml Isopropanol versetzt und anschliessend bei einem Wasserstoff-Druck von 80 bar, einer Temperatur von 80 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Ethandiamin, Wasser und Isopropanol entfernt wurden.

41.2 g dieser Reaktionsmischung wurden anschliessend bei 70 °C unter Vakuum destilliert, wobei 25.6 g Destillat bei einer Dampftemperatur von ca. 50 °C und 0.1 bar aufgefangen wurden. Erhalten wurde eine farblose Flüssigkeit mit einer Aminzahl von 802 mg KOH/g, einem AHEW von etwa 46.7 g/eq, einem RCI von 0.71, einer Viskosität von 3.2 mPa·s bei 20 °C und einem mittels GC bestimmten Gehalt an N-Furfuryl-1,2-ethandiamin von 94.6 Gewichts-% (Retentionszeit 7.3 min) und N-Tetrahydrofurfuryl-1,2-ethandiamin von 5.3 Gewichts-% (Retentionszeit 8.0 min), welche im Folgenden als **F-EDA** eingesetzt wurde.
¹H-NMR (CDCl₃): 7.33 (d, 1 H, Ar-H), 6.27 (m, 1 H, Ar-H), 6.14 (m, 1 H, Ar-H), 3.76 (s, 2 H, Ar-CH₂), 2.78 (m, 2 H, NHC*H*₂CH₂), 2.65 (m, 2 H, C*H*₂NH₂), 1.52 (br s, 3 H, NH und NH₂).
FT-IR: 3284, 3043, 2945, 2838, 1567, 1504, 1455, 1382, 1306, 1219, 1146,1108, 1073, 1009, 916, 883, 806, 738.

### N-Benzyl-1,2-ethandiamin (B-EDA):

180.3 g (3 mol) 1,2-Ethandiamin wurden bei Raumtemperatur vorgelegt, mit einer Lösung aus 106.0 g (1 mol) Benzaldehyd in 1200 ml Isopropanol vermischt und 2 Stunden gerührt, anschliessend bei 80 °C, 80 bar Wasserstoff-Druck und einem Fluss von 5 ml/min auf einer kontinuierlich arbeitenden Hydrierapparatur mit Pd/C-Festbettkatalysator hydriert und die hydrierte Lösung am Rotationsverdampfer bei 65 °C eingeengt, wobei unreagiertes 1,2-Ethandiamin, Wasser und Isopropanol entfernt wurden. Die so erhaltene Reaktionsmischung wurde bei 80°C unter Vakuum mittels Destillation gereinigt. Erhalten wurde eine farblose Flüssigkeit mit einem mittels GC bestimmten Gehalt an N-Benzyl-1,2-ethandiamin von > 97%.

### Herstellung von Mannichbasen:

### Mannichbase B-1:

In einem Rundkolben mit angehängter Kühlfalle (gefüllt mit Essigsäure 50 Gewichts-% in Wasser, Trockeneis im Kühlfinger, gekühlt im Eisbad) wurden 13.25 g (0.05 mol) 2,4,6-Tris(dimethylaminomethyl)phenol (Ancamine^{®} K54, von Evonik) unter Stickstoffatmosphäre vorgelegt und unter Rühren mit 22.03 g (0.15 mol) N-Furfuryl-1,2-ethandiamin (**F-EDA,** RCI 0.71, hergestellt wie vorgängig beschrieben) versetzt. Diese Reaktionsmischung wurde unter Rühren und Stickstoffstrom auf 140 °C geheizt, wobei freigesetztes Dimethylamin in der Kühlfalle aufgefangen wurde. Die Aminzahl der Reaktionsmischung sank innerhalb einiger Stunden von 735 mg KOH/g auf 576 mg KOH/g, worauf die Reaktionsmischung auf Raumtemperatur abgekühlt wurde. Erhalten wurde eine gelbliche klare Flüssigkeit enthaltend 2,4,6-Tris((2-furfurylaminoethyl)aminomethyl)phenol mit einer Viskosität bei 20 °C von 13.5 Pa·s, einem RCI von 0.5 und einem geschätzten AHEW von etwa 92 g/eq.

### Mannichbase B-2:

In einem Rundkolben mit angehängter Kühlfalle (gefüllt mit Essigsäure 50 Gewichts-% in Wasser, Trockeneis im Kühlfinger, gekühlt im Eisbad) wurden 13.25 g (0.05 mol) 2,4,6-Tris(dimethylaminomethyl)phenol (Ancamine^{®} K54, von Evonik) unter Stickstoffatmosphäre vorgelegt und unter Rühren mit 14.20 g (0.10 mol) N-Furfuryl-1,2-ethandiamin (**F-EDA,** RCI 0.71, hergestellt wie vorgängig beschrieben) und 6.00 g (0.025 mol) Polyoxypropylendiamin (Jeffamine^{®} D-230, mittleres Molekulargewicht 230 g/mol, von Huntsman) versetzt. Diese Reaktionsmischung wurde unter Rühren und Stickstoffstrom auf 140 °C geheizt, wobei freigesetztes Dimethylamin in der Kühlfalle aufgefangen wurde. Die Aminzahl der Reaktionsmischung sank innerhalb einiger Stunden von 677 mg KOH/g auf 524 mg KOH/g, worauf die Reaktionsmischung auf Raumtemperatur abgekühlt wurde. Erhalten wurde eine gelbliche klare Flüssigkeit mit einer Viskosität bei 20 °C von 6.2 Pa·s und einem geschätzten AHEW von etwa 105 g/eq.

### Mannichbase R-1:

Wie für die Mannichbase **B-1** beschrieben wurden 13.25 g (0.05 mol) 2,4,6-Tris-(dimethylaminomethyl)phenol (Ancamine^{®} K54, von Evonik) unter Stickstoffatmosphäre vorgelegt und unter Rühren mit 22.50 g (0.15 mol) N-Benzyl-1,2-ethandiamin (**B-EDA,** hergestellt wie vorgängig beschrieben) umgesetzt. Die Aminzahl der Reaktionsmischung sank innerhalb einiger Stunden von 705 mg KOH/g auf 565 mg KOH/g, worauf die Reaktionsmischung auf Raumtemperatur abgekühlt wurde. Erhalten wurde eine gelbliche klare Flüssigkeit mit einer Viskosität bei 20 °C von 44.2 Pa·s und einem geschätzten AHEW von etwa 96.7 g/eq.

Die Mannichbase **B-1** ist ein erfindungsgemässes Beispiel und enthält hauptsächlich eine Mannichbase der Formel (Ic). Die Mannichbase **B-2** ist ein erfindungsgemässes Beispiel und enthält neben einer Mannichbase der Formel (Ic) zusätzlich Mannichbasen der Formel (VI). Die Mannichbase **R-1** ist ein Referenzbeispiel und dient als Vergleich.

### Herstellung von Epoxidharz-Zusammensetzungen:

### Beispiele Z-1 bis Z-3 und Ref-1 bis Ref-3:

Für jedes Beispiel wurden die in der Tabelle 1 angegebenen Inhaltsstoffe der Harz-Komponente in den angegebenen Mengen (in Gewichtsteilen) mittels eines Zentrifugalmischers (SpeedMixer^{™} DAC 150, FlackTek Inc.) vermischt und unter Ausschluss von Feuchtigkeit aufbewahrt.

Ebenso wurden die der Tabelle 1 angegebenen Inhaltsstoffe der Härter-Komponente verarbeitet und aufbewahrt.

Anschliessend wurden die beiden Komponenten jeder Zusammensetzung mittels des Zentrifugalmischers zu einer homogenen Flüssigkeit verarbeitet und diese unverzüglich folgendermassen geprüft:
Die **Viskosität** wurde 5 min nach dem Vermischen der Harz- und der Härter-Komponente wie beschrieben bei einer Temperatur von 20 °C gemessen.

Die **Gelierzeit** wurde bestimmt, indem eine frisch vermischte Menge von ca. 3 g im Normklima mit einem Spatel in regelmässigen Abständen bewegt wurde, bis die Masse gelierte.

Die **Shore D** Härte wurde bestimmt nach DIN 53505 an zwei zylindrischen Prüfkörpern (Durchmesser 20 mm, Dicke 5 mm), wobei einer im Normklima und einer bei 8 °C und 80% relativer Feuchtigkeit gelagert und die Härte jeweils nach 1 Tag (24h) und nach 2 Tagen gemessen wurde.

Weiterhin wurde ein Film in einer Schichtdicke von 500 µm auf eine Glasplatte aufgezogen und dieser im Normklima gelagert bzw. ausgehärtet. An diesem Film wurde die **Königshärte** (Pendelhärte nach König nach DIN EN ISO 1522) nach 1 Tag, 2 Tagen, 7 Tagen und nach 14 Tagen bestimmt (**1d NK**), (**2d NK**), (**7d NK**), (**14d NK**). Nach 14 Tagen wurde der **Aspekt (NK)** des Films beurteilt. Als "schön" wurde ein klarer Film bezeichnet, welcher eine glänzende und nichtklebrige Oberfläche ohne Struktur aufwies. Als "Struktur" wird dabei jegliche Art von Zeichnung oder Muster auf der Oberfläche bezeichnet.

Ein weiterer Film wurde in einer Schichtdicke von 500 µm auf eine Glasplatte aufgezogen und dieser unmittelbar nach dem Applizieren während 7 Tagen bei 8 °C und 80% relativer Feuchtigkeit und anschliessend während 2 Wochen im Normklima gelagert, beziehungsweise ausgehärtet. 24 Stunden nach der Applikation wurde ein Flaschendeckel aus Polypropylen auf den Film aufgesetzt, unter welchem ein feuchter Schwamm platziert war. Nach weiteren 24 Stunden wurden der Schwamm und der Deckel entfernt und an einer neuen Stelle des Films platziert, wo sie nach 24 Stunden wieder entfernt und neu platziert wurden, insgesamt 4 mal. Anschliessend wurde der Aspekt dieses Films beurteilt (in den Tabellen mit **"Aspekt (8°/80%)"** bezeichnet), auf die gleiche Weise wie für den Aspekt (NK) beschrieben. Dabei wurde jeweils auch die Anzahl und Art der sichtbaren Marken angegeben, die im Film durch den feuchten Schwamm oder den aufgesetzten Deckel entstanden waren. Als "Blushing" wurde die Anzahl weiss verfärbte Flecken angegeben. Als "(1)" wurde ein schwacher, weiss verfärbter Fleck bezeichnet. Als "1" wurde ein deutlicher, weiss verfärbter Fleck bezeichnet. Als "Ring" wurde angegeben, ob ein ringförmiger Abdruck durch Einsinken des ersten, 24h nach Applikation aufgesetzten Deckels vorhanden war. Ein solcher ringförmiger Abdruck zeigt an, dass die Beschichtung noch nicht begehbar ist. Als "(ja)" wurde ein ganz leichter Abdruck, als "ja" ein deutlicher Abdruck durch Einsinken des ersten, 24h nach Applikation aufgesetzten Deckels angegeben. An den so ausgehärteten Filmen wurde wiederum die Königshärte bestimmt, jeweils nach 7 Tagen bei 8 °C und 80% relativer Feuchtigkeit (**Königsh. (7d 8°/80%)**), dann nach weiteren 2 Tagen im NK (**Königsh.** (**+2d NK**)) bzw. 7 Tagen im NK (**Königsh.** (**+7d NK**)) bzw. 14d im NK (**Königsh.** (**+14d NK**)).

Die Resultate sind in der Tabellen 1 angegeben.
Bei den Epoxidharz-Zusammensetzungen **Z-1** bis **Z-3** handelt es sich um erfindungsgemässe Beispiele. Bei den Epoxidharz-Zusammensetzungen **Ref-1** bis **Ref-3** handelt es sich um Vergleichsbeispiele.

**Tabelle 1: Zusammensetzung und Eigenschaften von Z-1 bis Z-3 und Ref-1 bis Ref-3. "n.b." steht für "nicht bestimmt"**

| **Beispiel** | | **Z-1** | **Ref-1** | **Z-2** | **Z-3** | **Ref-2** | **Ref-3** |
|---|---|---|---|---|---|---|---|
| **Harz-Komponente:** | | | | | | | |
| Araldite^{®} GY-250 | | 167.2 | 167.2 | 167.2 | 167.2 | 167.2 | 167.2 |
| Araldite^{®} DY-E | | 31.8 | 31.8 | 31.8 | 31.8 | 31.8 | 31.8 |
| **Härter-Komponente:** | | | | | | | |
| Mannichbase **B-1** | | 92.0 | - | - | 5.0 | - | - |
| Mannichbase **R-1** | | - | 96.7 | - | - | - | - |
| Mannichbase **B-2** | | - | - | 75.0 | - | - | - |
| **F-EDA** | | - | - | - | 46.7 | 46.7 | 46.7 |
| Ancamine^{®} K54 | | - | - | - | - | - | 5.0 |
| Viskosität (10') [Pa·s] | | 2.7 | 3.5 | 2.6 | 0.23 | 0.20 | 0.21 |
| Gelierzeit (h:min) | | 3:20 | 2:50 | 4:10 | 4:45 | 5:10 | 4:45 |
| Shore D | (1d NK) | 84 | 79 | 78 | 72 | 71 | 78 |
| | (2d NK) | 84 | 79 | 79 | 78 | 75 | 79 |
| Shore D | (1d 8°/80%) | 69 | n.b. | 30 | 13 | 7 | 40 |
| | (2d 8°/80%) | 71 | n.b. | 61 | 54 | 47 | 61 |
| Königshärte | (1d NK) | 137 | 197 | 112 | 66 | 48 | 88 |
| [s] | (2d NK) | 200 | 217 | 153 | 99 | 77 | 134 |
| | (7d NK) | 218 | | 192 | 148 | 122 | 169 |
| | (14d NK) | | | 200 | 160 | 148 | 167 |

| Aspekt (NK) | | schön | schön | schön | schön | schön | schön |
|---|---|---|---|---|---|---|---|
| Königsh. (7d 8°/80%) | | 113 | 160 | 60 | 18 | 11 | 31 |
| [s] | (+2d NK) | 136 | 204 | 111 | 38 | 27 | 62 |
| | (+7d NK) | 158 | | 140 | 71 | 41 | 87 |
| | (+14d NK) | 164 | | 148 | 91 | 70 | 99 |
| Aspekt (8°/80%) | | schön | schön | schön | schön | schön | schön |
| Blushing | | 0 | 0 | (1) | 1 | 1 | 1 |
| Ring | | kein | kein | kein | (ja) | ja | (ja) |

## Patentansprüche

1. Mannichbase der Formel (I), 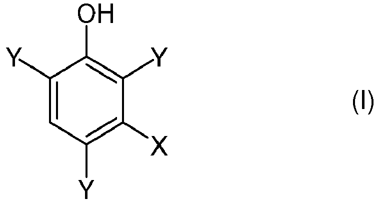 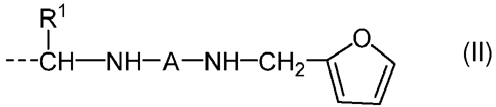 wobei
X für H oder einen linearen Kohlenwasserstoffrest mit 15 C-Atomen steht,
Y unabhängig voneinander jeweils für einen Rest der Formel (II) oder H oder Dimethylaminomethyl oder Methoxy steht, wobei mindestens einer der Reste Y für einen Rest der Formel (II) steht,
R¹ für H oder einen Alkylrest mit 1 bis 6 C-Atomen oder einen Phenylrest steht, und
A für einen Alkylenrest mit 2 bis 10 C-Atomen steht, wobei die beiden Stickstoffatome, an welche der Rest A gebunden ist, durch mindestens zwei C-Atome voneinander getrennt sind.

2. Mannichbase gemäss Anspruch 1, **dadurch gekennzeichnet, dass** R¹ für H steht.

3. Mannichbase gemäss einem der Ansprüche 1 oder 2, **dadurch gekennzeichnet, dass** A für einen linearen Alkylenrest mit 2 bis 6 C-Atomen steht, insbesondere für 1,2-Ethylen.

4. Mannichbase gemäss einem der Ansprüche 1 bis 3, **dadurch gekennzeichnet, dass** X für einen linearen Kohlenwasserstoffrest mit 15 C-Atomen steht, der Rest Y para zu X für einen Rest der Formel (II) steht, die weiteren Reste Y für H stehen und die Mannichbase somit die Formel (la) aufweist.

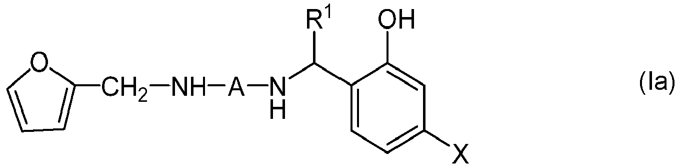

5. Mannichbase gemäss einem der Ansprüche 1 bis 3, **dadurch gekennzeichnet, dass** X für H steht, ein Rest Y ortho zur Phenolgruppe für Methoxy steht, die beiden weiteren Reste Y jeweils für einen Rest der Formel (II) stehen und die Mannichbase somit die Formel (Ib) aufweist.

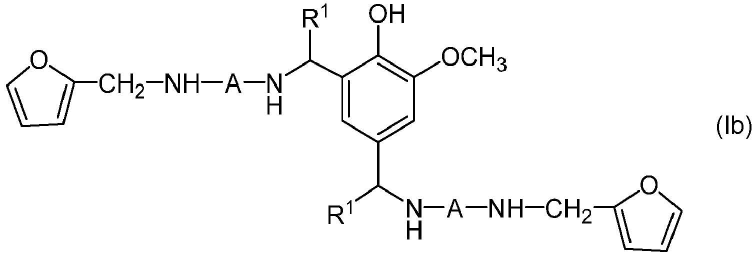

6. Mannichbase gemäss einem der Ansprüche 1 bis 3, **dadurch gekennzeichnet, dass** X für H steht, alle drei Reste Y für einen Rest der Formel (II) stehen und die Mannichbase somit die Formel (Ic) aufweist.

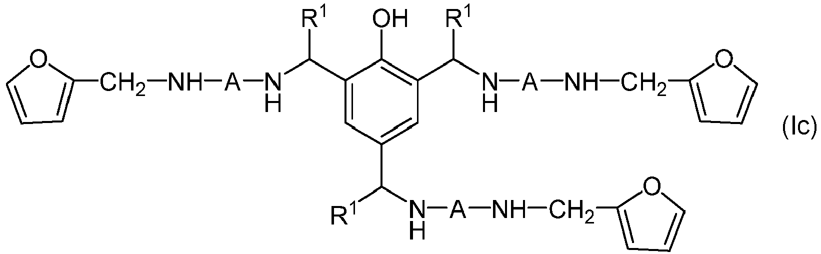

7. Mannichbase gemäss einem der Ansprüche 1 bis 3, **dadurch gekennzeichnet, dass** X für H steht, ein oder zwei Reste Y für einen Rest der Formel (II) mit R¹ = H stehen und die übrigen Reste Y jeweils für Dimethylaminomethyl stehen.

8. Mannichbase gemäss einem der Ansprüche 6 oder 7, **dadurch gekennzeichnet, dass** sie ein Reaktionsprodukt aus der Umaminierung von 2,4,6-Tris(dimethylaminomethyl)phenol mit mindestens einem Amin der Formel (IV) 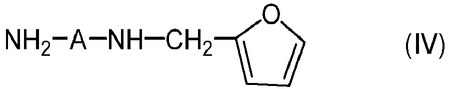 unter Freisetzung und Entfernung von Dimethylamin ist, wobei R¹ in Formel (Ic) für H steht.

9. Mannichbase gemäss Anspruch 8, **dadurch gekennzeichnet, dass** bei der Umaminierung von 2,4,6-Tris(dimethylaminomethyl)phenol mit mindestens einem Amin der Formel (IV) zusätzlich ein Polyetherdiamin, insbesondere ein Polyoxypropylendiamin mit einem mittleren Molekulargewicht Mₙ im Bereich von 200 bis 500 g/mol, mitverwendet wird.

10. Härter für Epoxidharze enthaltend mindestens eine Mannichbase gemäss einem der Ansprüche 1 bis 9.

11. Härter gemäss Anspruch 10, **dadurch gekennzeichnet, dass** mindestens ein weiterer Bestandteil ausgewählt aus weiteren Aminen, welche nicht der Formel (I) entsprechen, Beschleunigern und Verdünnern enthalten ist, insbesondere mindestens ein weiteres Amin, welches nicht der Formel (I) entspricht.

12. Härter gemäss Anspruch 11, **dadurch gekennzeichnet, dass** als weiteres Amin, welches nicht der Formel (I) entspricht, mindestens ein Amin der Formel (IV) enthalten ist, 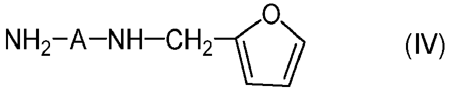 wobei A für einen Alkylenrest mit 2 bis 10 C-Atomen steht, wobei die beiden Stickstoffatome, an welche der Rest A gebunden ist, durch mindestens zwei C-Atome voneinander getrennt sind.

13. Epoxidharz-Zusammensetzung umfassend
- eine Harz-Komponente umfassend mindestens ein Epoxidharz und
- eine Härter-Komponente umfassend mindestens eine Mannichbase gemäss einem der Ansprüche 1 bis 9.

14. Epoxidharz-Zusammensetzung gemäss Anspruch 13, **dadurch gekennzeichnet, dass** die Härter-Komponente den Härter gemäss einem der Ansprüche 10 bis 12 umfasst.

15. Ausgehärtete Epoxidharz-Zusammensetzung erhalten aus der Epoxidharz-Zusammensetzung gemäss einem der Ansprüche 13 oder 14 nach dem Vermischen der Harz-Komponente und der Härter-Komponente.
